# EUROPÄISCHE PATENTSCHRIFT

(11) **EP 1 161 478 B1**
(45) Veröffentlichungstag und Bekanntmachung des Hinweises auf die Patenterteilung: **27.12.2006**
(21) Anmeldenummer: 99907613.6
(22) Anmeldetag: 09.03.1999
(51) Int. Cl.: C08G 61/00, H05B 33/14

(54) **TRIPTYCEN-POLYMERE UND -COPOLYMERE**
TRIPTYCENE POLYMERS AND COPOLYMERS
POLYMERES ET COPOLYMERES DE TRIPTYCENE

(43) Veröffentlichungstag der Anmeldung: 12.12.2001
(73) Patentinhaber: Covion Organic Semiconductors GmbH, 65926 Frankfurt (DE)
(72) Erfinder: BECKER, Heinrich, D-61479 Glashütten (DE); KREUDER, Willi, D-55126 Mainz (DE); SALBECK, Josef, D-34260 Kaufungen (DE); WEINFURTNER, Karl, Heinz, D-93057 Regensburg (DE)
(74) Vertreter: Benz, Jürgen
(86) Internationale Anmeldenummer: PCT/EP1999/001505
(87) Internationale Veröffentlichungsnummer: WO 2000/053655

(56) Entgegenhaltungen:
- EP-A- 0 581 058
- DE-A- 4 121 138
- DE-A- 19 744 792
- PATENT ABSTRACTS OF JAPAN vol. 017, no. 553 (P-1625), 5. Oktober 1993 & JP 05 158092 A (HITACHI LTD), 25. Juni 1993
- WASIELEWSKI ET AL: "High-quantum-yield long-lived charge separation in a photosynthetic reaction center model" JOURNAL OF THE AMERICAN CHEMICAL SOCIETY, Bd. 107, Nr. 19, 18. September 1985, Seite 5562/5563 XP002092033
- WASIELEWSKI ET AL: "Ultrafast photoinduced electron transfer in rigid donor-spacer -acceptor molecules: modification of spacer energetics as a probe for superexchang" TETRAHEDRON, Bd. 45, Nr. 15, 1. Januar 1989, Seiten 4785-4806, XP002092034

## Beschreibung

Die Erfindung betrifft konjugierte Polymere und Copolymere enthaltend Triptycenteilstrukturen.
Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), in Gebrauch.

Neben anorganischen sind seit etwa 30 Jahren auch niedermolekulare organische Elektrolumineszenzmaterialien und -vorrichtungen bekannt (siehe z.B. US 3,172,862). Bis vor kurzem waren aber solche Vorrichtungen in ihrer praktischen Verwendbarkeit stark eingeschränkt.

In WO 90/13148 und EP-A-0 443 861 sind Elektrolumineszenzvorrichtungen beschrieben, die einen Film aus einem konjugierten Polymer als lichtemittierende Schicht (Halbleiterschicht) enthalten. Solche Vorrichtungen bieten zahlreiche Vorteile wie die Möglichkeit, großflächige, flexible Displays einfach und kostengünstig herzustellen. Im Gegensatz zu Flüssigkristalldisplays sind Elektrolumineszenzdisplays selbstleuchtend und benötigen daher keine zusätzliche rückwärtige Beleuchtungsquelle.

Eine typische Vorrichtung nach WO 90/13148 besteht aus einer lichtemittierenden Schicht in Form eines dünnen, dichten Polymerfilms (Halbleiterschicht), der wenigstens ein konjugiertes Polymer enthält. Eine erste Kontaktschicht steht in Kontakt mit einer ersten Oberfläche, eine zweite Kontaktschicht mit einer weiteren Oberfläche der Halbleiterschicht. Der Polymerfilm der Halbleiterschicht hat eine genügend geringe Konzentration von extrinsischen Ladungsträgern, so daß beim Anlegen eines elektrischen Feldes zwischen den beiden Kontaktschichten Ladungsträger in die Halbleiterschicht eingebracht werden, wobei die eine Kontaktschicht positiv gegenüber der anderen wird und die Halbleiterschicht Strahlung aussendet. Die in solchen Vorrichtungen verwendeten Polymere sind konjugiert. Unter konjugiertem Polymer versteht man ein Polymer, das ein delokalisiertes Elektronensystem entlang der Hauptkette besitzt. Das delokalisierte Elektronensystem verleiht dem Polymer Halbleitereigenschaften und gibt ihm die Möglichkeit, positive und/oder negative Ladungsträger mit hoher Mobilität zu transportieren.

In WO 90/13148 wird als polymeres Material für die lichtemittierende Schicht Poly(p-phenylenvinylen) verwendet, und es wird vorgeschlagen, die Phenylgruppe in einem solchen Material durch ein heterocyclisches oder ein kondensiertes carbocyclisches Ringsystem zu ersetzen. Daneben wird auch Poly(p-phenylen), PPP, als elektrolumineszierendes Material verwendet (G. Grem et al., Synth. Met. 1992, 51, Seite 383).

Obwohl mit diesen Materialien gute Ergebnisse erzielt wurden, ist beispielsweise die Farbreinheit noch unbefriedigend. Weiterhin ist es mit den bisher bekannten Polymeren kaum möglich, eine blaue oder weiße Emission zu erzeugen.

Da zudem die Entwicklung von Elektrolumineszenzmaterialien, insbesondere auf Grundlage von Polymeren, noch in keiner Weise als abgeschlossen betrachtet werden kann, sind die Hersteller von Beleuchtungs- und Anzeigevorrichtungen an den unterschiedlichsten Elektrolumineszenzmaterialien für solche Vorrichtungen interessiert.

Dies liegt unter anderem auch daran, daß erst das Zusammenwirken der Elektrolumineszenzmaterialien mit den weiteren Bauteilen der Vorrichtungen Rückschlüsse auf die Qualität auch des Elektrolumineszenzmaterials zuläßt.

In der prioritätsälteren, nicht vorveröffentlichten, deutschen Patentanmeldung 197 44 792.9 ist die Verwendung von Triptycenderivaten als Elektrolumineszenzmaterial beschrieben. Gegenstand dieser Anmeldung sind die monomeren Triptycenderivate, die, um als Elektrolumineszenzmaterialien Verwendung zu finden, nach bekannten Methoden wie Eintauchen, Lackschleudern, Aufdampfen oder Auspuffern im Vakuum, in Form eines Films auf ein Substrat aufgebracht werden.

Aufgabe der vorliegenden Erfindung ist es, neue polymere Elektrolumineszenzmaterialien, die Triptycenteilstrukturen enthalten, bereitzustellen, die bei Verwendung in Beleuchtungs- oder Anzeigevorrichtungen geeignet sind, das Eigenschaftsprofil dieser Vorrichtungen zu verbessern.

Gelöst wurde die Aufgabe durch ein konjugiertes Polymer, enthaltend
a) 1 bis 100 mol-% mindestens einer Wiederholungseinheit WE1 der allgemeinen Formel (I),

   -B-Tr-A- (I)

   worin Tr ein Triptycenylenrest der allgemeinen Formel (II) 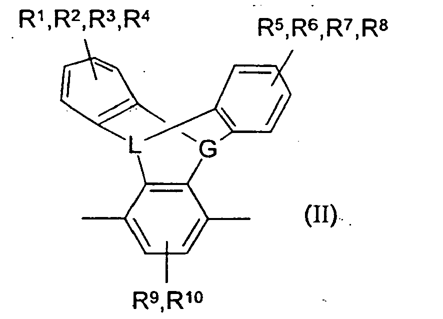 oder der allgemeinen Formel (III) 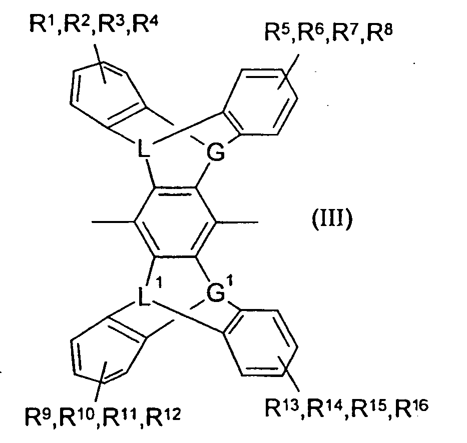 oder der allgemeinen Formel (IV) 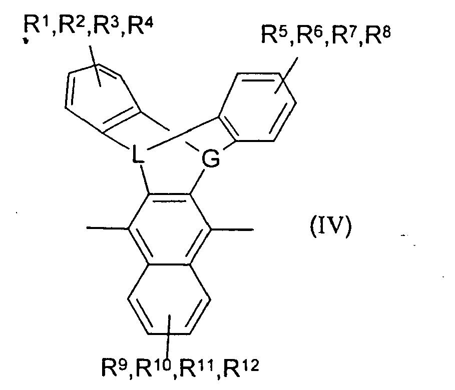 mit R¹ bis R¹⁶ = H, lineares oder verzweigtes C₁-C₂₂-Alkyl bzw. Alkoxy, wobei eine oder mehrere nicht benachbarte CH₂-Gruppen durch -O-, -S-, -CO-, -COO-, -O-CO-, eine Amino- oder Amidgruppe ersetzt sein können und wobei ein oder mehrere H-Atome durch F-Atome ersetzt sein können, C₆-C₂₀-Aryl bzw. Aryloxy, COOR, SO₃R, CN, Halogen oder NO₂,
   mit G, L, und gegebenenfalls G¹, L¹ = CR¹⁷, N, P, As, mit R¹⁷ = H, C₁-C₂₂-Alkyl bzw. Alkoxy, wobei eine oder mehrere nicht benachbarte CH₂-Gruppen durch -O-, -S-, -CO-, -COO-, -O-CO-, eine Amino- oder Amidgruppe ersetzt sein können und wobei ein oder mehrere H-Atome durch F-Atome ersetzt sein können, C₆-C₂₀-Aryl, Halogen oder CN bedeutet,
   A, B eine Einfachbindung, einen gegebenenfalls mit H, linearem oder verzweigtem C₁-C₂₂-Alkyl bzw. Alkoxy, wobei eine oder mehrere nicht benachbarte CH₂-Gruppen durch -O-, -S-, -CO-, -COO-, -O-CO-, eine Amino- oder Amidgruppe ersetzt sein können und wobei ein oder mehrere H-Atome durch F-Atome ersetzt sein können, C₆-C₂₀-Aryl bzw. Aryloxy, C₃-C₂₀-Heteroaryl, COOR, SO₃R, CN, Halogen, NO₂, Amino, Alkylamino oder Dialkylamino substituierten Vinylenrest, einen Ethinylenrest, einen Arylenrest der allgemeinen Formel (V) 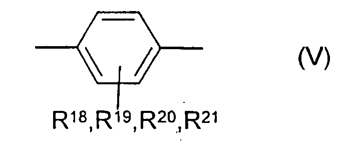 wobei R¹⁸ bis R²¹ die für R¹ bis R¹⁶ oben angegebene Bedeutung haben,
   einen Heteroarylenrest der allgemeinen Formel (VI) 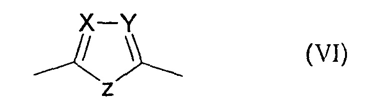 mit X, Y, = N, CR²², und Z = O, S, NR²³, CR²⁴R²⁵, CR²⁶=CR²⁷ oder CR²⁸=N-, wobei R²² bis R²⁸ die oben für R¹ bis R¹⁶ angegebene Bedeutung haben, oder einen Spirobifluorenylenrest der allgemeinen Formel (VII), 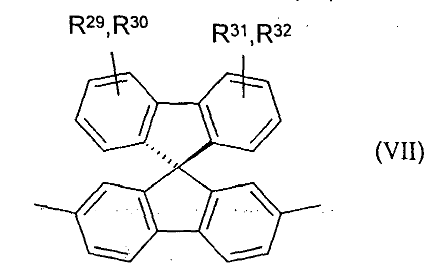 wobei R²⁹ bis R³² die oben für R¹ bis R¹⁶ angegebene Bedeutung haben, bedeuten und
b) 0 bis 99 mol-% mindestens einer Wiederholungseinheit WE2 der allgemeinen Formel (VIII) 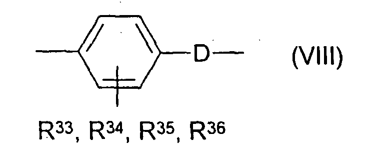
wobei R³³ bis R³⁶ die oben für R¹ bis R¹⁶ angegebene Bedeutung haben, oder der allgemeinen Formel (IX) 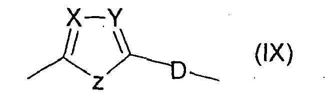 wobei X, Y und Z die oben angegebene Bedeutung haben und D eine Einfachbindung, einen gegebenenfalls mit H, linearem oder verzweigtem C₁-C₂2-Alkyl bzw. Alkoxy, wobei eine oder mehrere nicht benachbarte CH₂-Gruppen durch - O-, -S-, -CO-, -COO-, -O-CO-, eine Amino- oder Amidgruppe ersetzt sein können und wobei ein oder mehrere H-Atome durch F-Atome ersetzt sein können, C₆-C₂₀-Aryl bzw. Aryloxy, C₃-C₂₀-Heteroaryl, COOR, SO₃R, CN, Halogen, NO₂, Amino, Alkylamino oder Dialkylamino substituierten Vinylenrest oder einen Ethinylenrest bedeutet.

In einer bevorzugten Ausführungsform der Erfindung bedeuten L, G und gegebenenfalls L¹, G¹ eine CH-Gruppe.

A, B, bedeuten eine Einfachbindung, einen gegebenenfalls substituierten Vinylenrest, einen Ethinylenrest, einen gegebenenfalls substituierten Arylenrest, einen gegebenenfalls substituierten Heteroarylenrest oder einen Spirobifluorenylenrest.

Bevorzugte substituierte Vinylenreste sind Methylvinylen, Phenylvinylen, Cyanovinylen.

Besonders bevorzugt ist ein unsubstituierter Vinylenrest.

Bevorzugte Arylenreste sind 1,4-Phenylen, 2,5-Toluylen, 1,4-Naphthylen, 1,9 Antracenylen, 2,7-Phenantrylen, 2,7-Dihydrophenantrylen.

Bevorzugte Heteroarylenreste sind 2,5-Pyrazinylen, 3,6-Pyridazinylen, 2,5-Pyridinylen, 2,5-Pyrimidinylen, 2,5-Thiadiazolylen-(1,3,4), 2,4-Thiazolylen-(1,3), 2,5-Thiazolylen-(1,3), 2,4-Thiophenylen, 2,5-Thiophenylen, 2,4-Oxazolylen-(1,3), 2,5-Oxazolylen-(1,3) und 2,5-Oxadiazolylen-(1,3,4), 2,5-Indenylen, 2,6-Indenylen.

Methoden zur Synthese dieser Monomere beruhen z.B. auf der Synthese des 9,9'-Spirobifluorens, z.B. aus 2-Brombiphenyl und Fluorenon über eine Grignardsynthese, wie sie von R. G. Clarkson, M. Gomberg, J. Am. Chem. Soc. 1930, 52, Seite 2881 beschrieben ist, welches anschließend weiter in geeigneter Weise substituiert wird.

Funktionalisierungen von 9,9'-Spirobifluoren sind beispielsweise beschrieben in J. H. Weisburger, E. K. Weisburger, F. E. Ray, J. Am. Chem. Soc. 1959, 72, 4253; F. K. Sutcliffe, H. M. Shahidi, D. Paterson, J. Soc. Dyers Colour 1978, 94, 306; und G. Haas, V. Prelog, Helv. Chim. Acta 1969, 52, 1202.

Wesentlich günstiger erhält man das gewünschte Substitutionsmuster des 9,9'-Spirobifluoren-Monomers, wenn die Spiroverknüpfung bereits ausgehend von geeignet substituierten Edukten erfolgt, z.B. mit 2,7-difunktionalisierten Fluorenonen, und die noch freien 2',7'-Positionen nach Aufbau des Spiroatoms dann gegebenenfalls weiter funktionalisiert werden (z.B. durch Halogenierung oder Acylierung, mit anschließender C-C-Verknüpfung nach Umwandlung der Acetylgruppen in Aldehydgruppen, oder durch Heterocyclenaufbau nach Umwandlung der Acetylgruppen in Carbonsäuregruppen).

Die weitere Funktionalisierung kann nach an sich literaturbekannten Methoden erfolgen, wie sie in Standardwerken zur Organischen Synthese, z.B. Houben-Weyl, Methoden der Organischen Chemie, Georg-Thieme Verlag, Stuttgart und in den entsprechenden Bänden der Serie "The Chemistry of Heterocyclic Compounds" von
A. Weissberger und E. C. Taylor (Herausgeber) beschrieben sind.

Die substituierten Triptycen- bzw. Heterotriptycengrundkörper sind über verschiedene Synthesewege zugänglich. Exemplarisch, aber nicht einschränkend seien an dieser Stelle genannt:
1. Synthesen aus substituierten Anthracen (bzw. substituierten Acridin oder substituiertem Phenazin) und Dehydroaromaten z.B. ausgehend von
   a) substituierten o-Fluorbrombenzolen mit reaktiven Metallen wie z.B. Magnesium, z.B. analog zu G. Wittig, Org. Synth. IV 1963, 964;
   b) substituierten o-Dihalogenbenzolen und Butyllithum unter Metallhalogenideliminierung, z.B. analog zu H. Hart, S. Shamouilian, Y. Takehira J. Org. Chem. 46 (1981) 4427;
   c) substituierten Monohalogenbenzolen und starken Basen unter Halogenwasserstoffeliminierung, z.B. analog zu P. G. Sammes, D. J. Dodsworth, J. C. S. Chem. Commun. 1979, 33.
   d) substituierten Anthranilsäurederivaten und Isoamylnitril, z.B. analog zu C. W. Jefford, R. McCreadie, P. Müller, B. Siegfried, J. Chem. Educ. 48 (1971) 708.
   e) eine Übersicht zur Herstellung einer Reihe von substituierten Dehydroaromaten findet sich in Houben-Weyl, Methoden der Organischen Chemie, 4..Auflage 1981, Band V/2b, pp. 615, Georg-Thieme-Verlag, Stuttgart.
2. Synthesen durch Desaminierung substituierter Anthracen-9,10-imine z.B. analog zu L. J. Kricka, J. M. Vernon, J.C.S. Perkin I, 1973, 766.
3. Synthese durch Cycloaddition von substituierten 1,4-Chinonen mit substituierten Anthracenenderivaten z.B. analog zu E. Clar, Chem. Ber. 64 (1931) 1676; W. Theilacker, U. Berger-Brose, K. H. Beyer, Chem. Ber. 93 (1960) 1658; P. D. Bartlett, M. J. Ryan, J. Am. Chem. Soc. 64 (1942) 2649; P. Yates, P. Eaton, J. Am. Chem. Soc. 82 (1960) 4436. V. R. Skvarchenko, V. K. Shalaev, E. I. Klabunovskii, Russ. Chem. Rev. 43 (1974) 951;

Weitere Synthesen substituierter Triptycene finden sich exemplarisch in C. F. Wilcox, F. D. Roberts, J. Org. Chem. 30 (1965) 1959; T. H. Regan, J. B. Miller, J. Org. Chem. 32 (1967) 2798.

Weitere Synthesen für Heterotriptycene finden sich beispielsweise in D. Hellwinkel et al., Chem. Ber. 111 (1978); oder D. Hellwinkel et al., Angew. Chem. 24 (1969) 1049; N. P. McCleland et al., J. Am. Chem. Soc. (1927) 2753; N.A.A. AI-Jabar et al., J. Organomet. Chem. 287 (1985) 57.

Bistriptycengrundkörper bzw. Hetero-bistriptycengrundkörper sind ebenfalls über verschiedene Synthesewege zugänglich. Exemplarisch seien an dieser Stelle genannt:
1) Synthesen aus substituierten Anthracen (bzw. substituiertem Acridin oder substituiertem Phenazin) und substituierten Didehydrobenzolen z.B. analog zu H. Hart, S. Shamouilian, Y. Takehira J. Org. Chem. 46 (1981) 4427;
2) Synthese durch Cycloaddition von substituierten Anthracenenderivaten mit 1,4-Benzochninon z.B. analog zu E. Clar, Chem. Ber. 64 (1931) 1676; P. Yates, P. Eaton, J. Am. Chem. Soc. 82 (1960) 4436; W. Theilacker, U. Berger-Broske, K. H. Beyer, Chem. Ber. 93 (1960) 1658.

Weitere Synthesen finden sich exemplarisch in H. Hart et al., Tetrahedron 42 (1986) 1641; V. R. Skvarchenko et al., Russ. Chem. Rev. 43 (1974) 951; V. R. Skvarchenko et al., J. Org. Chem. USSR (engl. trans.) 3 (1967) 1477.

In einer bevorzugten Ausführungsform bestehen die erfindungsgemäßen Polymere aus genau einer Art von Wiederholungseinheiten WE1 (Homopolymere). Besonders bevorzugt sind Homopolymere, in denen A ausgewählt ist aus der Gruppe bestehend aus 2,5-Thiophenylen, 2,5-Oxadiazolylen, 1,4-Phenylen, Vinylen und Ethinylen, und B eine Einfachbindung ist.

Weiterhin bevorzugte Homopolymere sind solche, in denen A und B gleich sind und ausgewählt sind aus der Gruppe bestehend aus 2,5-Thiophenylen, 1,4-Phenylen, Vinylen und Ethinylen.

In einer weiterhin bevorzugten Ausführungsform enthalten die erfindungsgemäßen Polymere 1 bis 99 mol-% Wiederholungseinheiten WE2 (Copolymere). Bevorzugt enthalten die Copolymere 5 bis 95 mol% Wiederholungseinheiten WE2, besonders bevorzugt 10 bis 90 mol% Wiederholungseinheiten WE2.

Bevorzugte Copolymere sind ferner solche, in denen A eine Einfachbindung und B eine Einfachbindung, eine Vinylengruppe oder Ethinylengruppe bedeutet. Besonders bevorzugt sind solche Copolymere, in denen B eine Vinylengruppe bedeutet.

Bevorzugte Copolymere sind ferner binäre Copolymere aus Wiederholungseinheiten WE1 und Wiederholungseinheiten WE2 der allgemeinen Formel (VIII) oder (IX).

Weiterhin bevorzugte Copolymere sind ternäre Copolymere aus Wiederholungseinheiten WE1 und zwei Arten von Wiederholungseinheiten WE2 der allgemeinen Formel (VIII) oder (IX).

Besonders bevorzugte Copolymere sind solche, in denen die Wiederholungseinheiten WE2 Wiederholungseinheiten der allgemeinen Formel (VIII) sind.

Besonders bevorzugt sind ferner solche Copolymere, in denen die Gruppe D in den allgemeinen Formeln (VIII) und (IX) eine Vinylengruppe bedeutet.

Die Darstellung der Polymere erfolgt nach gängigen Methoden der Polyreaktionen, wie sie z.B. in "Makromoleküle" von Hans-Georg Elias (Hüthig & Wepf Verlag Basel-Heidelberg-New York) oder in Houben-Weyl, Methoden der organischen Chemie, Bd. XIV, Makromolekulare Stoffe (G. Thieme, Stuttgart 1961 u. 1963) beschrieben sind. Die Auswahl erfolgt dabei jeweils nach Art der Funktionalisierung der Monomere und gewünschter Molekularmasse.

Ausgehend von den wie beschrieben erhaltenen Monomeren ist die Polymerisation zu erfindungsgemäßen Polymeren nach mehreren Verfahren möglich.

Beispielsweise können Halogen-Derivate der Triptycene oxidativ (z.B. mit FeCl₃, siehe u.a. P. Kovacic et al., Chem. Ber., 87, 1987, 357 bis 379; M. Wenda et al., Macromolecules 25, 1992, 5125) oder elektrochemisch (siehe u.a. N. Saito et al., Polym. Bul. 30, 1993, 285) polymerisiert werden.

Erfindungsgemäße Polymere können ebenfalls aus Dihalogen-Derivaten durch Polymerisation unter Kupfer/Triphenylphosphan-Katalyse (s. z.B. G. W. Ebert et al., J. Org. Chem. 1988, 53, 4829 oder Nickel/Triphenylphosphan-Katalyse (siehe. z.B. H. Matsumoto et al., J. Org. Chem. 1983, 48, 840) hergestellt werden.

Aromatische Diboronsäuren und aromatische Dihalogenide oder aromatische Halogen-Boronsäuren lassen sich unter Palladiumkatalyse polymerisieren (Suzuki Kupplung) (s. z.B. M. Miyaura et al., Synth. Commun. 11, 1981, 513; R. B. Miller et al., Organometallics 3, 1984, 1261). In ähnlicher Weise lassen sich aromatische Distannane und aromatische Dihalogenide polymerisieren (s. z.B. J. K. Stille, Angew. Chem. Int. Ed. 25, 1986, 508).

Weiterhin können Dibromaromaten in Dilithio- oder Digrignardverbindungen überführt werden. Diese können dann mit weiterem Dihaloaromaten mittels CuCl₂ polymerisiert werden (s.z.B. G. Wittig et al., Liebigs Ann. Chem. 704, 91, 1967; H. A. Stabb et al., Chem. Ber. 100, 1967, 293 und T. Kaufmann, Angew. Chem. 86, 1974,321).
Besondere Methoden sind zur Herstellung von ebenfalls erfindungsgemäßen Poly-(triptycenylvinylenen) erforderlich. So kann die Synthese z.B. durch Polykondensation von para-Dihalogenmethyl substituierten Triptycenderivaten erfolgen. Die Polymerisation erfolgt hierbei in einem geeigneten Lösungsmittel durch Zugabe von Base (s. z.B. H.Hörhold et al., Makromol. Chem, Macromol. Symp. 12, 1987, 229-258). Ebenfalls möglich ist eine Precursorpolymerisation; hierbei wird ein Poly-(triptycenylenvinylen) durch Eliminierung eines vorhandenen Precursorrestes (z.B. CH₂S⁺R₂) durch Temperaturbehandlung oder Basenbehandlung hergestellt (s. z.B. R. A. Wessling, J. Polym. Sci; Polym. Sym. 72, 1985, 55-66).

Weitere Möglichkeiten zur Herstellung von Poly-(triptycenylenen) sind z.B. Hornerpolymerisation und Wittigpolymerisation. Hierbei werden zwei Monomertypen (Aldehyde mit Phosphonaten (Horner Polymerisation); Aldehyde mit Triarylalkylphosphoniumsalzen (Wittig Polymerisation)) unter Zusatz einer Base polymerisiert. Allgemein sind diese Herstellungsverfahren z.B. in DD 84272, H. Hörhold et al., Makromol. Chem, Macromol. Symp. 12, 1987, 229-258 und H. Hörhold et al., Z. Chem. 27, 1987, 126 beschrieben.

Cyanosubstituierte Poly-(triptycenylvinylene) können durch Knoevenagel-Reaktion hergestellt werden. Hierbei wird ein Bis-Cyanomethyl substituierter Aromat unter Basenzusatz mit einem Dialdehyd umgesetzt (s. z.B. H. Hörhold et al., Plaste und Kautschuk 17, 1970, 84).

Zur Herstellung von Copolymeren können Triptycen- bzw. Heterotriptycenmonomere zusammen mit einem oder mehreren Comonomeren polymerisiert werden, wie beispielsweise in "Makromoleküle" von Hans-Georg Elias (Hüthig & Wepf Verlag Basel-Heidelberg-New York), S. 32-40, beschrieben.

Die Aufarbeitung der erfindungsgemäßen Polymeren kann nach bekannten, dem Fachmann geläufigen Methoden, wie sie beispielsweise bei D. Braun, H. Cherdron, W. Kern, Praktikum der makromolekularen organischen Chemie, 3. Aufl. Hüthig Verlag, Heidelberg, 1979, S. 87-89 oder R. J. Young, P. A. Lovell, Introduction to Polymers, Chapman & Hall, London 1991 beschrieben sind, erfolgen.
Beispielsweise kann man die Reaktionsmischung filtrieren, mit wäßriger Säure verdünnen, extrahieren und das nach Trocknen und Abziehen des Lösungsmittels erhaltene Rohprodukt durch Umfällen aus geeigneten Lösungsmitteln durch Zugabe von Fällungsmitteln weiter reinigen. Nachfolgend können noch polymeranaloge Reaktionen zur weiteren Funktionalisierung des Polymeren vorgenommen werden. So können z.B. endständige Halogenatome durch Reduktion mit z.B. LiAlH₄ reduktiv entfernt werden (siehe z.B. J. March, Advanced Organic Chemistry, 3. Aufl. McGraw-Hill, s. 510).

Die erfindungsgemäßen Polymere eignen sich zur Verwendung als Elektrolumineszenzmaterialien.

Als Elektrolumineszenzmaterialien im Sinne der Erfindung gelten Materialien, die als oder in einer aktiven Schicht in einer Elektrolumineszenzvorrichtung Verwendung finden. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht). Darüber hinaus ist auch die Verwendung als Elektronenblockierschicht oder Lochblockierschicht erfindungsgemäße Anwendung.

Gegenstand der Erfindung ist daher auch die Verwendung der erfindungsgemäßen Polymere als Elektrolumineszenzmaterial. Ferner ist Gegenstand der Erfindung ein Elektrolumineszenzmaterial, welches die erfindungsgemäßen Polymere enthält.

Um als Elektrolumineszenzmaterialien Verwendung zu finden, werden die erfindungsgemäßen Polymere im allgemeinen nach bekannten, dem Fachmann geläufigen Methoden, wie Eintauchen (Dipping), Lackschleudern (Spin-coating), Aufdampfen oder Auspuffern im Vakuum, in Form eines Films auf ein Substrat aufgebracht.

Gegenstand der Erfindung ist ebenfalls eine Elektrolumineszenzvorrichtung mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Ladungstransportschicht und/oder eine Ladungsinjektionsschicht sein.
Der allgemeine Aufbau solcher Elektrolumineszenzvorrichtungen ist beispielsweise in US 4,539,507 und US 5,151,629 beschrieben.

Sie enthalten üblicherweise eine elektrolumineszierende Schicht zwischen einer Kathode und einer Anode, wobei mindestens eine der Elektroden zumindest für einen Teil des sichtbaren Spektrums transparent ist. Zusätzlich können zwischen der elektrolumineszierenden Schicht und der Kathode eine oder mehrere Elektroneninjektions- und/oder Elektronentransportschichten eingebracht sein und/oder zwischen der elektrolumineszierenden Schicht und der Anode eine oder mehrere Lochinjektions- und/oder Lochtransportschichten eingebracht sein. Als Kathode können vorzugsweise Metalle oder metallische Legierungen, z.B. Ca, Mg, Al, In, Mg/Ag dienen. Als Anode können Metalle, z.B. Au, oder andere metallisch leitende Stoffe, wie Oxide, z.B: ITO (Indiumoxid/Zinnoxid) auf einem transparentem Substrat, z.B. aus Glas oder einem transparenten Polymer, dienen.

Im Betrieb wird die Kathode auf negatives Potential gegenüber der Anode gesetzt. Dabei werden Elektronen von der Kathode in die Elektroneninjektionsschicht/Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendeten Materialien variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplern.

Verbindungen der Formel (1) eignen sich weiterhin beispielsweise zur Verwendung in optischen Speichern, als photorefraktive Materialien, für nichtlinear-optische (NLO) Anwendungen, als optische Aufheller und Strahlungskonverter und, bevorzugt, als Lochtranspörtmaterialien in photovoltaischen Zellen, wie sie z.B. in WO-A 97/10 617 und DE-A 197 11 713 beschrieben, auf die für diese Anwendungen verwiesen wird.

Die erfindungsgemäßen Polymere sind hervorragend in organischen Lösungsmitteln löslich. Die Filmbildungseigenschaften sind im Vergleich mit Poly(p-phenylen) hervorragend. Besonders hervorzuheben ist die Temperaturstabilität der Emissionsfarbe, d.h. daß die Morphologie des Polymeren nicht thermisch aktiviert zerstört wird. Weiterhin werden hohe Ladungsträgermobilitäten beobachtet.

Die Erfindung wird durch die nachstehenden Beispiele näher erläutert, ohne sie dadurch zu beschränken.

### Beispiele

Die Darstellung von Polymer-LEDs erfolgte nach dem im folgenden skizzierten allgemeinen Verfahren. Dieses mußte natürlich im Einzelfall auf die jeweiligen Gegebenheiten (z.B. Polymerviskosität, optimale Schichtdicke des Polymers im Device, u.ä.) angepaßt werden. Die im nachfolgenden beschriebenen LEDs sind jeweils Einschichtsysteme, d.h. Substrat//ITO//Polymer//Kathode.

Allgemeines Verfahren zur Herstellung von hocheffizienten, langlebigen LEDs unter Verwendung von triptycenhaltigen Polymeren:

Nachdem man die ITO-beschichteten Substrate (z.B. Glasträger, PET-Folie) auf die richtige Größe zugeschnitten hatte, wurden sie in mehreren Reinigungsschritten im Ultraschallbad gereinigt (z.B. Seifenlösung, Millipore-Wasser, Isopropanol).
Zur Trocknung wurden sie mit einer N₂-Pistole abgepustet und in einem Exsikkator gelagert. Vor der Beschichtung mit dem Polymer wurde sie mit einem Ozon-Plasma-Gerät für ca. 20 Minuten behandelt. Von dem jeweiligen Polymer wurde eine Lösung (in der Regel mit einer Konzentration von 4-25 mg/ml in beispielsweise Toluol, Chlorbenzol, Xylol:Cyclohexanon (4:1)) angesetzt und durch Rühren bei Raumtemperatur gelöst. Je nach Polymer kann es auch vorteilhaft sein, für einige Zeit bei 50 - 70 C zu rühren. Hatte sich das Polymer vollständig gelöst, wurde es durch einen 5µm Filter filtriert und bei variablen Geschwindigkeiten (400-6000 U/min) mit einem Spin-coater aufgeschleudert. Die Schichtdicken können dadurch im Bereich von ca. 50 und 300 nm variiert wurden.
Auf die Polymerfilme wurden anschließend Elektroden aufgebracht. Dies geschah in der Regel durch thermisches Verdampfen (Balzer BA360 bzw. Pfeifer PL S 500). Anschließend wurde die durchsichtige ITO-Elektrode als Anode und die Metallelektrode (z.B. Ca) als Kathode kontaktiert und es wurden die Device-Parameter bestimmt.

### Beispiel M1: Synthese von Dihydrotriptycen-1,4-chinon

17,8 g (100 mmol) Anthracen und 10,8 g (100 mmol) p-Benzochinon (frisch sublimiert) wurden unter Stickstoff bei 135 C in 200 ml *p*-Xylol gelöst. Nach einigen Minuten begann sich aus der nun rot gefärbten Lösung ein gelber, kristalliner Niederschlag abzuscheiden. Nach 4 Stunden ließ man auf Raumtemperatur abkühlen und saugte den Niederschlag ab. Der gelbe Feststoff wurde mit *p*-Xylol nachgewaschen und im Vakuum getrocknet. Die erhaltenen 26,0 g (91 mmol, 91% Ausbeute) wurden in 100 ml *p*-Xylol unter N₂ auf 130 C erhitzt und diese Temperatur 0,5 h lang gehalten, auf Raumtemperatur abgekühlt, abgesaugt, mit Methanol nachgewaschen und getrocknet. Man erhielt 23,5 g (82 mmol, 82% Ausbeute) Dihydrotriptycen-1,4-chinon als fahlgelbe Kristalle.
Schmelzpunkt: 232 C
¹H NMR: (400 MHz; CDCl₃): [ppm] = 3.15 (t, 2H, tert. H), 4,86 (s, 2H, Enyl-H), 6,30 (s, 2H, Brückenkopf-H) 7,07 und 7,39 (4H, m, *J* = 5,3 Hz, 2,3 Hz-Phenyl-H), 7,17-7,20 ppm, m, 4H, Phenyl-H).

### Beispiel M2: Synthese von 1,4-Triptycen-1,4-chinon

37,0 g (129 mmol) Dihydrotriptycen-1,4-chinon wurden in 350 ml Eisessig suspendiert und in der Siedehitze mit 1,5 ml HBr (48%ig in Wasser) versetzt. Es wurde zwei Stunden zum Rückfluß erhitzt. In der Siedehitze wurde dann eine Lösung von 13,0g KlO₃ (60 mmol) innerhalb von 5 Minuten zugetropft. Sofort zeigt sich eine Gelbfärbung der Suspension. Man ließ erkalten, setzte bei 50 C noch 200 ml Wasser zu und saugte ab. Dann wurde mehrmals mit Na₂SO₃-Lösung, anschließend mehrmals mit Wasser gewaschen und im Vakuum getrocknet. Das Rohprodukt (35,2g, 96% Ausbeute) wurde zweimal je eine Stunde in 150 ml Isopropanol trituriert. Man erhielt 30,9 g (108,7 mmol, 84 % Ausbeute) 1,4-Triptycen-1,4-chinon als leuchtend gelbe pulvrige Substanz.
Schmelzpunkt: 273-275 C
¹H NMR: (400 MHz; CDCl₃): [ppm] = 5,79 (s, 2H, Brückenkopf-H), 6,59 (s, 2H, Enyl-H), 7,03 und 7,42 (m, 8H, *J* = 2,3 Hz, 5,3 Hz, AB-System Phenyl-H).

### Beispiel M3: Synthese von 1,4-Dihydroxy-1,4-dimethyltriptycen

In einem 1 l Vierhalskolben wurden 148 ml (237 mmol, 2,7 eq) einer 1,6 M Lösung von Methyllithium in Diethylether mit 300 ml THF (destilliert von Na/Benzophenon) vorgelegt und auf-78 C gekühlt (Aceton/Trockeneis). Gleichzeitig wurde eine Lösung von 25,0 g (87,9 mmol) 1,4-Triptycen-1,4-chinon in 600 ml THF auf dieselbe Temperatur abgekühlt. Die Lösung des 1,4-Triptycen-1,4-chinons wurde in einen Tropftrichter überführt, der mittels Trockeneis zusätzlich gekühlt wurde. Unter kräftigem Rühren wurde die Eduktlösung langsam zugetropft (1 Stunde), wobei sich die Lösung sofort blau bis blaugrün färbte. Nach beendeter Zugabe wurde die Temperatur noch eine weitere Stunde gehalten und anschließend die Kühlung entfernt. Man ließ auf Raumtemperatur erwärmen und rührte über Nacht.
Die Suspension wurde im Vakuum auf etwa 200 ml eingeengt und anschließend auf eine Mischung aus 1,4 L Eiswasser auf 10 g NH₄Cl gegossen. Beim Eingießen trat Wärmeentwicklung auf und es fiel ein hellbeiger Niederschlag aus, der sich beim Erwärmen auf Raumtemperatur verflüssigte. Das resultierende Öl wurde abgetrennt und die Wasserphase wurde dreimal mit 500 ml CH₂Cl₂ extrahiert. Die vereinigten organische Phase wurden zweimal mit je 200 ml Wasser gewaschen, mit Na₂SO₄ getrocknet und so weit wie möglich einrotiert.

Es verblieb eine braune, zähe Masse, die mit 30 ml Diethylether/Hexan 2:1 solange im Ultraschallbad behandelt wurde, bis alles Öl in Lösung gegangen war und sich ein weißer Niederschlag gebildet hatte. Der Niederschlag wurde abgesaugt und die Mutterlauge wurde erneut einrotiert und auf die gleiche Art und Weise behandelt, wobei die Volumina der Et₂O/Hexan-Mischung jedes Mal etwas kleiner gewählt wurden. Der Vorgang wurde wiederholt, bis kein Niederschlag mehr ausfiel. Zur weiteren Reinigung wurde das Reaktionsgemisch in Diethylether refluxiert, auf 20 C gekühlt und abgesaugt. Es wurden 14,9 g (47,1 mmol, 54%) 1,4-Dihydroxy-1,4-dimethyltriptycen als weißes Pulver erhalten.
¹H NMR: (400 MHz; DMSO-d₆): =1,09 (s, 6H, Methyl-H); 4,84 (s, 2H, Hydroxy-H); 5,34 (s, 2H, Chinon-H); 5,63 (s, 2H, Brückenkopf-H); 6,90, 6,92, 7,28, 7,33 (m, je 2H, J=5,3 Hz und 2,3 Hz, Phenyl-H).
¹H NMR: (400 MHz; CDCl₃): =1,27 (s, 6H, Methyl-H); 1,63 (s, 2H, Hydroxy-H); 5,39 (s, 2H, Chinon-H); 5,54 (s, 2H, Brückenkopf-H); 6,91 (m, 2H, J=5,5 Hz und 2,3 Hz, Phenyl-H); 6,95 m, 2H, J=5,3 Hz und 2,0 Hz, Phenyl-H); 7,32 (m, 4H, J=5,3 Hz, 2,3 Hz und 2,0 Hz, Phenyl-H).

### Beispiel M4: Synthese von 1,4-Dimethyltriptycen

6,70 g (53,3 mmol, 2,1 eq) SnCl₂·2 H₂O wurden in 200 ml 50%iger Essigsäure gelöst. Dazu wurde eine methanolische Lösung von 8,44 g (25,7 mmol) 1,4-Dihydroxy-1,4-dimethyltriptycen so langsam zugetropft, daß die Temperatur nicht höher als max. 45 C anstieg. Die Reaktionslösung nahm eine gelbliche Färbung an und ein weißer Niederschlag schied sich ab. Nach erfolgter Zugabe wurde weitere 2h bei Raumtemperatur gerührt. Danach kühlte man auf -18 C und saugte den erhaltenen Niederschlag ab, wusch ihn mit ca. 1 L Wasser säurefrei und trocknete im Vakuum. Die erhaltene Mutterlauge wurde danach etwas einrotiert und der nach erneutem Abkühlen ausgefallenen Niederschlag wiederum abgesaugt. Es wurde 7,0 g Rohprodukt erhalten. Die Verbindung wurde in etwa 300 ml Aceton in der Siedehitze gelöst und anschließend mit 50 ml Wasser gefällt. Die Lösung wurde im Eisfach gekühlt und der Niederschlag wurde abgesaugt. Nach erneuter Durchführung der Prozedur erhielt man 5,20 g (18,4 mmol, 72%) 1,4-Dimethyltriptycen als weiße Kristallflitter.
Schmelzpunkt: 246-249 C
¹H NMR: (400 MHz; DMSO-d₆): =2,43 (s, 6H, Methyl-H); 5,80 (s, 2H, Brückenkopf-H); 6,71 (s, 2H, Phenyl-H); 6,98 und 7,45 (m, 8H, J = 2,3 Hz, 5,3 Hz, AB System, Phenyl-H).
¹H NMR: (400 MHz; CDCl₃₎: =2,46 (s, 6H, Methyl-H); 5,64 (s, 2H, Brückenkopf-H); 6,70 (s, 2H, Phenyl-H); 6,97 und 7,36 (m, 8H, *J* = 2,3 Hz, 5,3 Hz, AB-System, Phenyl-H).

### Beispiel M5: Synthese von 1,4-Bis(brommethyl)triptycen

5,20 g (18,4 mmol) 1,4-Dimethyltriptycen wurden in 150 ml trockenem Tetrachlorkohlenstoff gelöst, mit 3,45 g (19,3 mmol) N-Bromsuccinimid, sowie 0,20 g (1,22 mmol) Diazoisobutyronitril versetzt. Die Suspension wurde unter Bestrahlung mit Licht zum gelinden Rückfluß erhitzt. Man ließ eine Stunde reagieren. Nach Kontrolle im DC (Hexan/CH₂Cl₂1:1) wurde solange N-Bromsuccinimid zugegeben, bis der Spot zwischen Edukt und Produkt verschwunden war. Danach ließ man erkalten und trennte durch Filtration vom Succinimid ab. Die Reaktionslösung wurde weitestgehend (30 ml) einrotiert, mit wenig Hexan versetzt und gekühlt. Der Niederschlag wurde abgesaugt und getrocknet. Es wurden 7,70 g (17,5 mmol, 95%) fahlgelbes Rohprodukt erhalten. Zur Reinigung kristallisierte man aus Eisessig um. Es wurden 5,6 g (12,7 mmol, 70%) 1,4-Bis(brommethyl)triptycen als farblose Kristalle erhalten.
Schmelzpunkt: 198-208 C
¹H NMR: (400 MHz; CDCl₃): = 4,67 (s, 4H, Brommethyl-H); 5,40 (s, 2H, Brückenkopf-H), 6,90 (s, 2H, Phenyl-H); 7,02 und 7,47 (m, J=5,3Hz, 3,3Hz, 8H, AB-System, Phenyl-H).

### Beispiel P1: Copolymer aus 80% 2,5-Bis(chlormethyl)-1-methoxy-4-(3,7-dimethyloctyloxy)benzol und 20% 1,4-Bis(brommethyl)triptycen (Polymer 1):

In einem trockenen 2 L Vierhalskolben mit mechanischem Teflonrührer, Rückflußkühler, Thermometer und Tropftrichter wurden 720 ml trockenes und O₂-freies 1,4-Dioxan auf 95 C erhitzt. Dann wurde eine Lösung von 2,89 g (8 mmol) 2,5-Bis(chlormethyl)-1-methoxy-4-(3',7'-dimethyloctyloxy)benzol und 880 mg (2 mmol) 1,4-Bis(brommethyl)triptycen in 10 ml trockenem 1,4-Dioxan zugesetzt. Nun wurde eine Lösung von 2,92 g (26 mmol) Kalium-terf butylat in 25 ml trockenem 1,4-Dioxan innerhalb von 5 Minuten zu der intensiv gerührten Mischung getropft. Die Farbe veränderte sich dabei von farblos über gelb nach orangerot. Nach 5 Minuten wurden weitere 2,24 g (20,0 mmol) Kalium-*tert*-butylat, gelöst in 20 ml 1,4-Dioxan, zugegeben. Nach 2 Stunden Rühren bei 95-97 C wurde auf 55 C abgekühlt und ein Gemisch aus 4 ml Essigsäure und 4 ml 1,4-Dioxan zugesetzt. Die nun orangefarbene Lösung wurde auf 1 L intensiv gerührtes Wasser gegossen. Das ausgefallene Polymer wurde durch Filtration durch einen Polypropylenfilter isoliert und im Vakuum getrocknet. Die Rohausbeute betrug 2,50 g (8,7 mmol, 87%).
Das Polymer wurde unter Erhitzen auf 60 C in 330 ml THF gelöst und durch Zusatz von 330 ml Methanol bei 40 C gefällt. Nach Trocknen im Vakuum wurde dieser Schritt wiederholt. Man erhielt nach Trocknung im Vakuum 1,46 g (= 5,10 mmol, 51 %) des Polymer 1 als hell-orangefarbene Fasern.
Der Gehalt an Triptycen-Gruppen wurde durch ¹H-NMR-Spektroskopie bestimmt. Hierzu wurde das Signal der Triptycen Brückenkopf-H-Atome (6,0 ppm) integriert und mit dem OCH₃ und OCH₂ Signale bei 4,2-3,6 ppm verglichen; es wurden 9% Triptycen-Einheiten im Polymer bestimmt.
¹H-NMR (400 MHz, CDCl₃): (ppm) = 7,9-6,6 (breites Multiplett, 5,6 H; Aryl-H, Olefin-H); 6,0 (breites Singulett; 0,4 H; Triptycen-Brückenkopf-H); 4,2-3,6 (br. m [?], 4 H; OCH₂, OCH₃); 2,0-0,9 (breiter Multiplett, 9,6 H; aliphatische Seitenkette); 0,89, 0,86 (2 Singuletts, 7,2 H; 3 x CH₃).
GPC: THF + 0,25% Oxalsäure; Säulensatz SDV500, SDV1000, SDV10000 (Fa. PSS), 35 C, UV-Detektion 254 nm, Polystyrol-Standard: M_{w} = 3,0 10⁵ g/mol, Mₙ = 4,5 10⁴ g/mol.
Elektrolumineszenzmessung: 0,34% maximale Quanteneffizienz bei 5,2V, eine Leuchtdichte von 100 cd/m² wurde bei 6,81 V, 15,07 mA/cm² erreicht.

### Beispiel P2: Copolymer aus 91% 2,5-Bis(chlormethyl)-1-methoxy-4-(3,7-dimethyloctyloxy)benzol und 9% 1,4-Bis(brommethyl)triptycen (Polymer 2):

In einem trockenen 2 L Vierhalskolben mit KPG-Rührer, Rückflußkühler, Thermometer und Tropftrichter wurden 1000 ml trockenes und O₂-freies 1,4-Dioxan auf 88-90 C erhitzt. Dann wurde eine Lösung von 4,34 g (12 mmol) 2,5-Bis(chlormethyl)-1-methoxy-4-(3',7'-dimethyloctyloxy)benzol und 528 mg (1,2 mmol) 1,4-Bis(brommethyl)triptycen in 20 ml trockenem 1,4-Dioxan zugesetzt. Unter intensivem Rühren wurde dann eine Lösung von 3,85 g (34,3 mmol) Kalium-*tert-*butylat in 34 ml trockenem 1,4-Dioxan innerhalb von 5 Minuten zu Reaktionsmischung getropft. Die Farbe veränderte sich dabei von farblos über gelb nach orangerot. Nach 5 Minuten wurden weitere 3,85 g (34,3 mmol) Kalium-*tert-*butylat, gelöst in 26 ml 1,4-Dioxan, zugegeben. Nach 2 Stunden Rühren bei 88 C wurde auf 55 C abgekühlt und mit 12 ml einer Mischung 1,4-Dioxan/Eisessig 1:1 versetzt. Die nun orangefarbene viskose Lösung wurde auf 1 L intensiv gerührtes Wasser gegossen. Das ausgefallene Polymer wurde durch Filtration durch einen Polypropylenfilter isoliert und im Vakuum getrocknet. Es wurden 3,4 g rohes Polymer erhalten.
Das Polymer wurde unter Erhitzen auf 60 C in 450 ml THF gelöst und durch Zusatz von 560 ml Methanol bei einer Temperatur <40 C gefällt. Nach Trocknen im Vakuum wurde dieser Schritt wiederholt. Man erhielt nach Trocknung im Vakuum 2,60 g (= 9,03 mmol, 68%) des Polymers 2 als hell-orangefarbene Fasern.
Der Gehalt an Triptycen-Gruppen wurde durch ¹H-NMR-Spektroskopie bestimmt. Hierzu wurde das Signal der Triptycen Brückenkopf-H (6,0 ppm) integriert und mit dem OCH₃ und OCH₂ Signale bei 4,2-3,6 ppm verglichen; es wurden 3,5% Triptycen-Einheiten im Polymer bestimmt.
¹H-NMR (400 MHz, CDCl₃): (ppm) = 7,9-6,6 (breites Multiplett, 4 H; Aryl-H, Olefin-H); 6,0 (breites Singulett; zus. 0,02 H; Triptycen-Brückenkopf-H); 4,2-3,6 (br. m, 5 H; OCH₂, OCH₃); 2,0-0,9 (breites Multiplett, 10 H; aliphatische Seitenkette); 0,89, 0,86 (2 Singuletts, 9 H; 3 x CH₃). GPC: THF + 0,25% Oxalsäure; Säulensatz SDV500, SDV1000, SDV10000 (Fa. PSS), 35 C, UV-Detektion 254 nm, Polystyrol-Standard:
M_{w} = 2,0 10⁵ g/mol, Mₙ = 3,1 10⁴ g/mol.
Elektrolumineszenzmessung: 0,21 % maximale Quanteneffizienz bei 5,2 V, eine Leuchtdichte von 100 cd/m² wurde bei 5,05 V, 11,17 mA/cm² erreicht.

### Copolymer aus 80% 2,5-Bis(chlormethyl)-3'-(3,7-dimethyloctyloxy)biphenyl und 20% 1,4-Bis(brommethyl)triptycen (Polymer 3):

In einem trockenen 2 L Vierhalskolben mit mechanischem Rührer, Rückflußkühler, Thermometer und Tropftrichter wurden 0,72 kg trockenes und O₂-freies 1,4-Dioxan vorgelegt und unter Rühren auf 98 C erhitzt. Dann wurde eine Lösung 3,26 g (8 mmol) 2,5-Bis(chlormethyl)-3'(3,7-dimethyloctyloxy)biphenyl und 0,88 g (2 mmol) 1,4-Bis(brommethyl)triptycen, gelöst in 30 ml trockenem 1,4-Dioxan, zugesetzt. Nun wurde eine Lösung von 2,87 g (26 mmol, 2,6 Äquivalente) Kalium-*tert*-butylat in 26 ml trockenem 1,4-Dioxan innerhalb von 5 Minuten zu der intensiv gerührten Mischung getropft. Die Farbe veränderte sich dabei von farblos über grün nach hell-orange; die Viskosität der Lösung nahm leicht zu. Nach 5 Minuten Rühren bei 98 C wurden nochmals 2,24 g (20 mmol, 2,0 Äquivalente) Kalium-*tert*-butylat in 20 ml 1,4-Dioxan innerhalb von einer Minute zugesetzt. Nach weiteren 2 Stunden Rühren bei 95 -98 C wurde auf 50 C abgekühlt und ein Gemisch aus 4 ml Essigsäure und 4 ml 1,4-Dioxan zugesetzt. Das Polymer wurde nach 20 Minuten Nachrühren durch Zusetzen der Reaktionslösung zu 0,7 L intensiv gerührtem Wasser ausgefällt. Das so erhaltene Polymer wurde abfiltriert und zweimal mit je 100 ml Methanol gewaschen. Nach Vakuumtrocknung bei Raumtemperatur wurden 3,17 g (9,8 mmol, 98%) rohes Polymer 3 erhalten.
Das Rohprodukt wurde unter Erhitzen auf 60 C in 400 ml THF gelöst und durch Zusatz von 400 ml Methanol gefällt. Nach Trocknen im Vakuum und Waschen mit 100 ml Methanol wurde dieser Schritt wiederholt. Man erhielt nach 2-tägiger Trocknung im Vakuum 1,84 g (= 5,7 mmol, 57%) des Polymer 3 als hell-orangefarbene Fasern.
Der Gehalt an Triptycen-Gruppen wurde durch ¹H-NMR-Spektroskopie bestimmt. Hierzu wurde das Signal der Triptycen Brückenkopf-H (5,9 ppm) integriert und mit dem OCH₂ Signal bei 4,0 ppm verglichen; es befanden sich 14% Triptycen-Einheiten im Polymer.
¹H-NMR (400 MHz, CDCl₃): (ppm) = 7,9-6,1 (breites Multiplett, 9,2 H; Aryl- und Olefin-H); 5,9 (breites Singulett; 0,28 H; Triptycen-Brückenkopf-H); 4,0 (breites Singulett, 1,6 H); 1,95-0,85 (breites Multiplett, 15,2 H; aliphatische H). GPC: THF + 0,25% Oxalsäure; Säulensatz SDV500, SDV1000, SDV10000 (Fa. PSS), 35 C, UV-Detektion 254 nm, Polystyrol-Standard: M_{w} = 4,4 10⁵ g/mol, Mₙ = 9,1 10⁴g/mol.
Elektrolumineszenzmessung: 0,47% maximale Quanteneffizienz bei 10,7 V, eine Leuchtdichte von 100 cd/m2 wurde bei 10,9 V erreicht. ₘₐₓ=517 nm.

### Beispiel P4: Copolymer aus 75% 2,5-Bis(chlormethyl)-1-methoxy-4-(3,7-dimethyloctyloxy)benzol und 25% 1,4-Bis(brommethyl)triptycen (Polymer 4):

In einem trockenen 500 ml Vierhalskolben mit mechanischem Teflonrührer, Rückflußkühler, Thermometer und Tropftrichter wurden 385 ml trockenes und O₂-THF vorgelegt. Dann wurden 1,58 g (3,6 mmol) 2,5-Bis(brommethyl)-1-methoxy-4-(3',7'-dimethyloctyloxy)benzol und 528 mg (1,2 mmol) 1,4-Bis(brommethyl)triptycen zugesetzt. Nun wurde eine Lösung von 1,32 g (11,8 mmol) Kalium-*tert*-butylat in 12 ml trockenem THF innerhalb von 5 Minuten zu der intensiv gerührten Mischung getropft. Die Farbe veränderte sich dabei von farblos über gelb nach orangerot. Nach 5 Minuten wurden weitere 1,1 g (9,8 mmol) Kalium-*tert*-butylat, gelöst in 10 ml THF auf einmal zugegeben. Nach 2 Stunden Rühren bei Raumtemperatur wurde für eine Stunde auf 60 C erhitzt und ein Gemisch aus 2 ml Essigsäure und 2 ml 1,4-Dioxan zugesetzt. Die nun orange Lösung wurde auf 1 L intensiv gerührtes Wasser gegossen. Das ausgefallene Polymer wurde durch Filtration durch einen Polypropylenfilter isoliert und im Vakuum getrocknet. Die Rohausbeute betrug 1,2g. Das Polymer wurde unter Erhitzen auf 60 C in 160 ml THF gelöst und durch Zusatz von 200 ml Methanol bei Raumtemperatur gefällt. Nach Trocknen im Vakuum wurde dieser Schritt wiederholt. Man erhielt nach Trocknung im Vakuum 0,89 g (= 2,98 mmol, 62%) des Polymer 4 als hell-orange Fasern.
Der Gehalt an Triptycen-Gruppen wurde durch ¹H-NMR-Spektroskopie bestimmt. Hierzu wurde das Signal der Triptycen Brückenkopf-H (6,0 ppm) integriert und mit dem OCH₃ und OCH₂ Signale bei 4,2-3,6 ppm verglichen; es wurden 15% Triptycen-Einheiten im Polymer bestimmt.
¹H-NMR (400 MHz, CDCl₃): (ppm) = 7,9-6,6 (breites Multiplett, 4 H; Aryl-H, Olefin-H); 6,0 (breites Singulett; 0,3 zusätzliche H; Triptycen-Brückenkopf-H); 4,2-3,6 (breiter Multiplett, 5 H); OCH₂, OCH₃); 2,0-0,9 (breites Multiplett, 10 H; aliphatische Seitenkette); 0,89, 0,86 (2 Singuletts, 9 H; 3 x CH₃).
GPC: THF + 0,25% Oxalsäure; Säulensatz SDV500, SDV1000, SDV10000 (Fa. PSS), 35 C, UV-Detektion 254 nm, Polystyrol-Standard: M_{w} = 3,0 10⁵ g/mol, Mₙ = 4,5 10⁴ g/mol.
Elektrolumineszenzmessung: 0,96% maximale Quanteneffizienz bei 6,01 V, eine Leuchtdichte von 100 cd/m² wurde bei 4,11 V/16,07 mA/cm² erreicht.

## Patentansprüche

1. Konjugiertes Polymer, enthaltend
a) 1 bis 100 mol-% mindestens einer Wiederholungseinheit WE1 der allgemeinen Formel (I),
-B-Tr-A- (I)
worin Tr ein Triptycenylenrest der allgemeinen Formel (II) 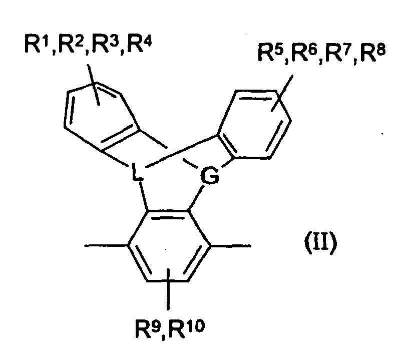 oder der allgemeinen Formel (III) 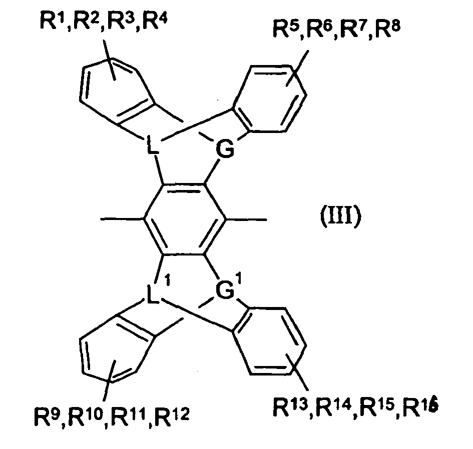 oder der allgemeinen Formel (IV) 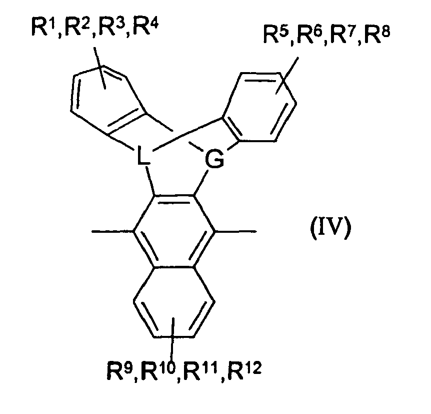 mit R¹ bis R¹⁶ = H, lineares oder verzweigtes C₁-C₂₂-Alkyl bzw. Alkoxy, wobei eine oder mehrere nicht benachbarte CH₂-Gruppen durch -O-, -S-, -CO-, -COO-, -O-CO-, eine Amino- oder Amidgruppe ersetzt sein können und wobei ein oder mehrere H-Atome durch F-Atome ersetzt sein können, C₆-C₂₀-Aryl bzw. Aryloxy, COOR, SO₃R, CN, Halogen oder NO₂,
mit G, L, und gegebenenfalls G¹, L¹ = CR¹⁷ , N, P, As, mit R¹⁷ = H, C₁-C₂₂-Alkyl bzw. Alkoxy, wobei eine oder mehrere nicht benachbarte CH₂-Gruppen durch -O-, -S-, -CO-, -COO-, -O-CO-, eine Amino- oder Amidgruppe ersetzt sein können und wobei ein oder mehrere H-Atome durch F-Atome ersetzt sein können, C₈-C₂₀-Aryl, Halogen oder CN bedeutet,
A, B eine Einfachbindung, einen gegebenenfalls mit H, linearem oder verzweigtem C₁-C₂₂-Alkyl bzw. Alkoxy, wobei eine oder mehrere nicht benachbarte CH₂-Gruppen durch -O-, -S-, -CO-,
-COO-, -O-CO-, eine Amino- oder Amidgruppe ersetzt sein können und wobei ein oder mehrere H-Atome durch F-Atome ersetzt sein können, C₆-C₂₀-Aryl bzw. Aryloxy, C₃-C₂₀-Heteroaryl, COOR, SO₃R, CN, Halogen, NO₂, Amino, Alkylamino oder Dialkylamino substituierten Vinylenrest, einen Ethinylenrest, einen Arylenrest der allgemeinen Formel (V) 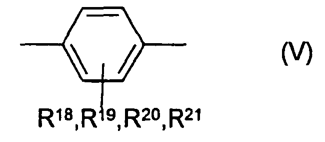 wobei R¹⁸ bis R²¹ die für R¹ bis R¹⁶ oben angegebene Bedeutung haben,
einen Heteroarylenrest der allgemeinen Formel (VI) 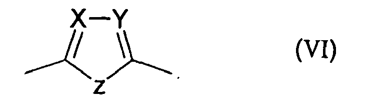 mit X, Y, = N, CR²², und Z = O, S, NR²³, CR²⁴R²⁵, CR²⁶=CR²⁷ oder CR²⁸=N-, wobei R²² bis R²⁸ die oben für R¹ bis R¹⁶ angegebene Bedeutung haben, oder einen Spirobifluorenylenrest der allgemeinen Formel (VII), 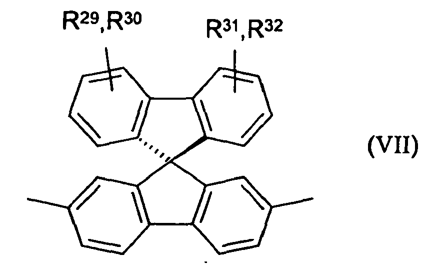 wobei R²⁹ bis R³² die oben für R¹ bis R¹⁶ angegebene Bedeutung haben, bedeuten
und
b) 0 bis 99 mol-% mindestens einer Wederholungseinheit WE2 der allgemeinen Formel (VIII) 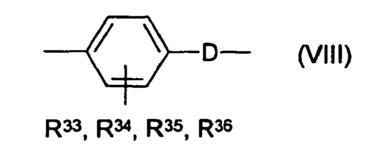
wobei R³³ bis R³⁶ die oben für R¹ bis R¹⁶ angegebene Bedeutung haben, oder der allgemeinen Formel (IX) 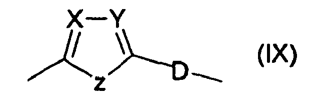 wobei X, Y und Z die oben angegebene Bedeutung haben und D eine Einfachbindung, einen gegebenenfalls mit H, linearem oder verzweigtem C₁-C₂₂-Alkyl bzw. Alkoxy, wobei eine oder mehrere nicht benachbarte CH₂-Gruppen durch -O-, -S-, -CO-, -COO-, -O-CO-, eine Amino- oder Amidgruppe ersetzt sein können und wobei ein oder mehrere H-Atome durch F-Atome ersetzt sein können, C₆-C₂₀-Aryl bzw. Aryloxy, C₃-C₂₀-Heteroaryl, COOR, SO₃R, CN, Halogen, NO₂, Amino, Alkylamino oder Dialkylamino substituierten Vinylenrest oder einen Ethinylenrest bedeutet.

2. Polymer nach Anspruch 1, worin L, G und gegebenenfalls L¹, G¹ eine CH-Gruppe bedeuten.

3. Polymer nach Anspruch 1 oder 2, das ein Homopolymer aus Wiederholungseinheiten WE1 ist.

4. Polymer nach Anspruch 3, worin A ausgewählt ist aus der Gruppe bestehend aus 2,5-Thiophenylen, 2,5-Oxadiazolylen, 1,4-Phenylen, Vinylen und Ethinylen, und B eine Einfachbindung ist.

5. Polymer nach Anspruch 4, worin A und B gleich sind und ausgewählt sind aus der Gruppe bestehend aus 2,5-Thiophenylen, 1,4-Phenylen, Vinylen und Ethinylen.

6. Polymer nach Anspruch 1 oder 2, enthaltend 1 bis 99 mol-% Wiederholungseinheiten WE2.

7. Polymer nach Anspruch 6, worin A eine Einfachbindung und B eine Einfachbindung, eine Vinylengruppe oder Ethinylengruppe bedeutet.

8. Polymer nach Anspruch 7, worin B eine Vinylengruppe bedeutet.

9. Polymer nach einem der Ansprüche 6 bis 8, das ein binäres Copolymer aus Wiederholungseinheiten WE1 und Wiederholungseinheiten WE2 der allgemeinen Formel (VIII) oder (IX) ist.

10. Polymer nach einem der Ansprüche 6 bis 8, das ein temäres Copolymer aus Wiederholungseinheiten WE1 und zwei Arten von Wiederholungseinheiten WE2 der allgemeinen Formel (VIII) oder (IX) ist.

11. Polymer nach einem der Ansprüche 9 oder 10, wobei die Wiederholungseinheiten WE2 Wiederholungseinheiten der allgemeinen Formel (VIII) sind.

12. Polymer nach einem der Ansprüche 9 bis 11, worin D eine Vinylengruppe bedeutet.

13. Verwendung von Polymeren nach einem der Ansprüche 1 bis 12 als Elektrolumineszenzmaterial.

14. Elektrolumineszenzmaterial, enthaltend ein Polymer nach einem der Ansprüche 1 bis 12.

15. Verfahren zur Herstellung eines Elektrolumineszenzmaterials, bei dem ein Polymer gemäß einem der Ansprüche 1 bis 12 in Form eines Films auf ein Substrat aufgebracht wird.

16. Elektrolumineszenzvorrichtung mit einer oder mehreren aktiven Schichten, wobei mindestens eine dieser aktiven Schichten ein Polymer gemäß einem der Ansprüche 1 bis 12 enthält.

## Claims

1. A conjugated polymer containing
a) from 1 to 100 mol% of at least one recurring unit RU1 of the general formula (I)
-B-Tr-A- (I)
in which Tr is a triptycenylene radical of the general formula (II) 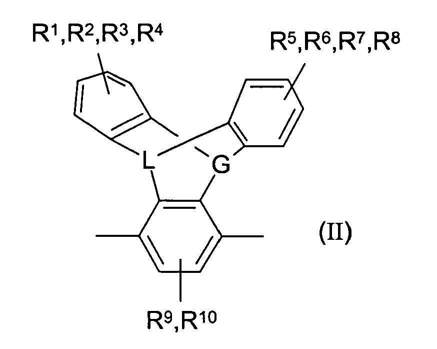 or of the general formula (III) 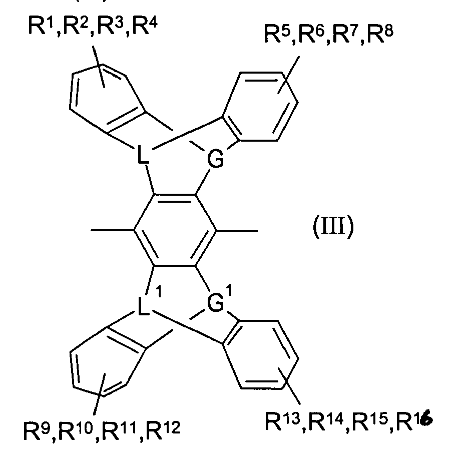 or of the general formula (IV) 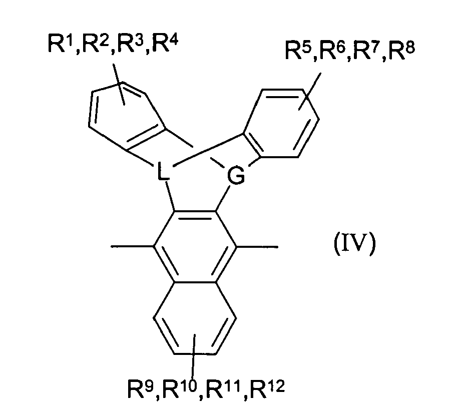 where R¹ to R¹⁶ = H, linear or branched C₁-C₂₂-alkyl or alkoxy, in which one or more non-adjacent CH₂ groups may be replaced by -O-, -S-, -CO-, -COO-, -O-CO-, an amino or amide group and in which one or more H atoms may be replaced by F atoms, or C₆-C₂₀-aryl or aryloxy, COOR, SO₃R, CN, halogen or NO₂,
where G, L and where appropriate G¹ and L¹ = CR¹⁷ , N, P, As, where R¹⁷ = H, C₁-C₂₂-alkyl or alkoxy, in which one or more non-adjacent CH₂ groups may be replaced by -O-, -S-, -CO-, -COO-, -O-CO-, an amino or amide group and in which one or more H atoms may be replaced by F atoms, or C₆-C₂₀-aryl, halogen or CN,
A and B are a single bond, a vinylene radical which is optionally substituted by H, linear or branched C₁-C₂₂-alkyl or alkoxy, in which one or more non-adjacent CH₂ groups may be replaced by -O-, -S-, -CO-, -COO-, -O-CO-, an amino or amide group and in which one or more H atoms may be replaced by F atoms, or C₆-C₂₀-aryl or aryloxy, C₃-C₂₀-heteroaryl, COOR, SO₃R, CN, halogen, NO₂, amino, alkylamino or dialkylamino, or are an ethynylene radical, an arylene radical of the general formula (V) 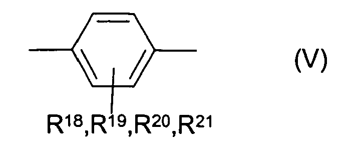 where R¹⁸ to R²¹ are as defined above for R¹ to R¹⁶,
a heteroarylene radical of the general formula (VI) 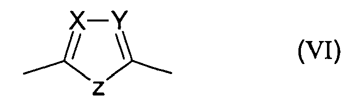 where X and Y = N or CR²², and Z = O, S, NR²³, CR²⁴R²⁵, CR²⁶=CR²⁷ or CR²⁸=N-, in which R²² to R²⁸ are as defined above for R¹ to R¹⁶, or a spirobifluorenylene radical of the general formula (VII) 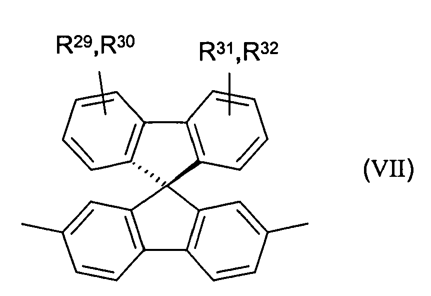 where R²⁹ to R³² are as defined above for R¹ to R¹⁶,
and
b) from 0 to 99 mol% of at least one recurring unit RU2 of the general formula (VIII) 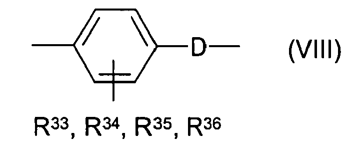
where R³³ to R³⁶ are as defined above for R¹ to R¹⁶, or of the general formula (IX) 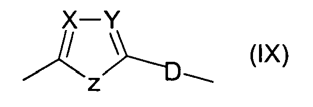 where X, Y and Z are as defined above, and D is a single bond, a vinylene radical which is optionally substituted by H, linear or branched C₁-C₂₂-alkyl or alkoxy, in which one or more non-adjacent CH₂ groups may be replaced by -O-, -S-, -CO-, -COO-, -O-CO-, an amino or amide group and in which one or more H atoms may be replaced by F atoms, or C₆-C₂₀-aryl or aryloxy, C₃-C₂₀-heteroaryl, COOR, SO₃R, CN, halogen, NO₂, amino, alkylamino or dialkylamino, or is an ethynylene radical.

2. Polymer as claimed in claim 1, in which L, g and, where appropriate, L¹ and G¹ are a CH group.

3. Polymer as claimed in claim 1 or 2, which is a homopolymer comprising recurring units RU1.

4. Polymer according to claim 3, in which A is selected from the group consisting of 2,5-thiophenylene, 2,5-oxadiazolylene, 1,4-phenylene, vinylene and ethynylene, and B is a single bond.

5. Polymer according to claim 4, in which A and B are identical and are selected from the group consisting of 2,5-thiophenylene, 1,4-phenylene, vinylene and ethynylene.

6. Polymer according to claim 1 or 2, comprising from 1 to 99 mol% of recurring units RU2.

7. Polymer according to claim 6, in which A is a single bond and B is a single bond, a vinylene group or an ethynylene group.

8. Polymer according to claim 7, in which B is a vinylene group.

9. Polymer according to one of claims 6 to 8, which is a binary copolymer comprising recurring units RU1 and recurring units RU2 of the general formula (VIII) or (IX).

10. Polymer according to one of claims 6 to 8, which is a ternary copolymer comprising recurring units RU1 and two types of recurring units RU2 of the general formula (VIII) or (IX).

11. Polymer according to one of claims 9 or 10, where the recurring units RU2 are recurring units of the general formula (VIII).

12. Polymer according to one of claims 9 to 11, in which D is a vinylene group.

13. Use of polymers according to one of claims 1 to 12 as electroluminescent material.

14. Electroluminescent material comprising a polymer as claimed in one of claims 1 to 12.

15. Process for the preparation of an electroluminescent material in which a polymer as claimed in one of claims 1 to 12 is applied in the form of a film to a substrate.

16. Electroluminescent device having one or more active layers, where at least one of these active layers comprises a polymer as claimed in one of claims 1 to 12.

## Revendications

1. Polymère conjugué, contenant
a) au moins 1 à 100% d'unités molaires d'une unité répétée WE1 avec la formule générale (I)
-B-Tr-A- (I)
où Tr signifie un reste triptycenylène avec la formule générale (II) 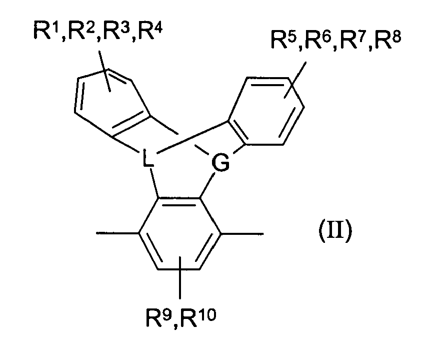 ou avec la formule générale (III) 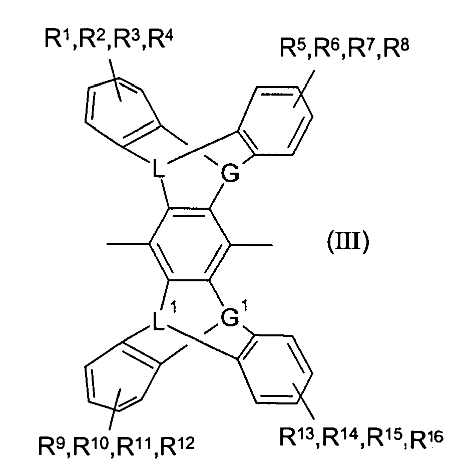 ou avec la formule générale (IV) 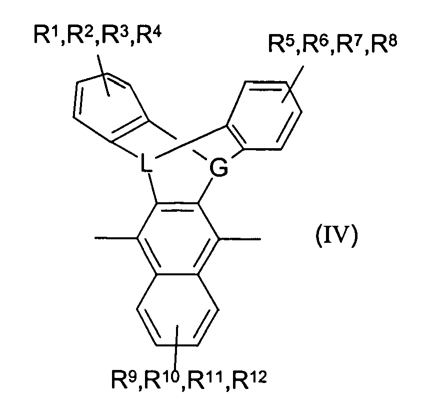 avec R¹ à R¹⁶ = H, alkyle linéaire ou ramifié C₁-C₂₂ respectivement alkoxy, où l'un ou plusieurs groupements CH₂ non voisinés peuvent être remplacés par -O-, -S-, -CO-, -COO-, -O-CO-, un groupement amino ou amido et où l'un ou plusieurs atomes de H peuvent être remplacés par les atomes de F, aryle C₆-C₂₀ respectivement aryloxy, COOR, SO₃R, CN, halogène ou NO₂, avec G, L et dans les conditions données les G¹, L¹ = CR¹⁷, N, P, As avec R¹⁷ = H, alkyle C₁-C₂₂ respectivement alkoxy, où l'un ou plusieurs groupements CH₂ non voisinés peuvent être remplacés par -O-, -S-, -CO-, -COO-, -O-CO-, un groupement amino ou amido et où l'un ou plusieurs atomes de H peuvent être remplacés par les atomes de F, C₆-C₂₀, halogène ou CN, A, B signifie une liaison simple, dans les conditions données avec H, l'alkyle linéaire ou ramifié C₁-C₂₂ respectivement alkoxy, où l'un ou plusieurs groupements CH₂ non voisinés peuvent être remplacés par -O-, -S-, -CO-, -COO-, -O-CO-, un groupement amino ou amido et où l'un ou plusieurs atomes de H peuvent être remplacés par les atomes de F; aryle respectivement aryloxy C₆-C₂₀, hétéroaryle C₃-C₂₀, COOR, SO₃R, CN, halogène, NO₂, des restes de vinylène substitués avec amino, alkylamino ou dialkylamino, un reste éthinylène, un reste arylène avec la formule générale (V) 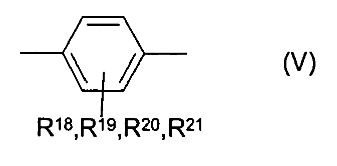 où R¹⁸ à R²¹ ont la signification indiquée ci-dessus pour R¹ à R¹⁶, un reste hétéroarylène avec la formule générale (VI) 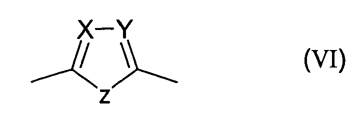 avec X, Y=N, CR²², et Z=O, S, NR²³, CR²⁴R²⁵, CR²⁶=CR²⁷ où CR²⁸=N-, où R²² à R²⁸ ont la signification indiquée ci-dessus pour R¹ à R¹⁶, où un reste spirobifluorenylène avec la formule générale (VII) 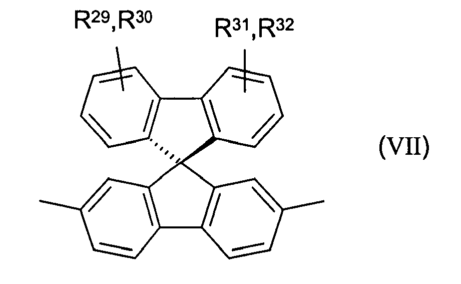 où R²⁹ à R³² ont la signification indiquée ci-dessus pour R¹ à R¹⁶. et
b) au moins 0 à 99% d'unités molaires d'une unité répétée WE2 avec la formule générale (VIII) 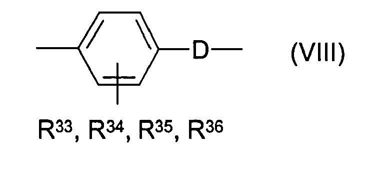
où R³³ à R³⁶ ont la signification indiquée ci-dessus pour R¹ à R¹⁶ avec la formule générale (IX) 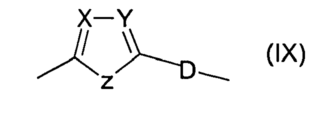 où X, Y, Z ont la signification indiquée ci-dessus pour et D signifie une liaison simple, dans les conditions données avec H, alkyle linéaire ou ramifié C₁-C₂₂ respectivement alkoxy, où l'un ou plusieurs groupements CH₂ non voisinés peuvent être remplacés par -O-, -S-, -CO-, -COO-, -O-CO-, un groupement amino ou amido et où l'un ou plusieurs atomes de H peuvent être remplacés par les atomes de F; aryle respectivement aryloxy C₆-C₂₀, hétéroaryle C₃-C₂₀, COOR, SO₃R, CN, halogène, NO₂, des restes de vinylène substitués avec amino, alkylamino ou dialkylamino, un reste éthinylène.

2. Polymère selon la revendication 1, où L, G et dans les conditions données L¹, G¹ signifient un groupement CH-.

3. Polymère selon la revendication 1 ou 2, qui est un homopolymère à unités répétées WE1.

4. Polymère selon la revendication 3, où A est du groupement composé de 2,5-thiophenylène, 2,5-oxadiazolylène, 1,4-phenylène, vinylène et ethinylène, et B est une liaison simple.

5. Polymère selon la revendication 4, où A et B sont identiques et sont choisis du groupement composé de 2,5-thiophenylène, 1,4-phenylène, vinylène et ethinylène.

6. Polymère selon la revendication 1 ou 2, qui contient 1 à 99% unités molaires répétées WE2.

7. Polymère selon la revendication 6, où A signifie une liaison simple et B une liaison simple, un groupement vinylène ou un groupement ethinylène.

8. Polymère selon la revendication 7, où B signifie un groupement vinylène.

9. Polymère selon l'une des revendications 6 à 8, qui est un copolymère binaire des unités répétées WE1 et des unités répétées WE2 avec la formule générale (VIII) ou (IX).

10. Polymère selon l'une des revendications 6 à 8, qui est un copolymère ternaire des unités répétées WE1 et des unités répétées WE2 avec la formule générale (VIII) ou (IX).

11. Polymère selon l'une des revendications 9 ou 10, où les unités répétées WE2 sont les unités répétées avec la formule générale (VIII).

12. Polymère selon l'une des revendications 9 à 11, où D représente un groupement vinylène.

13. Utilisation des polymères selon l'une des revendications 1 à 12 comme matériel électroluminiscent.

14. Matériel électroluminiscent contenant un polymère selon l'une des revendications 1 à 12.

15. Procédé pour obtenir un matériel électroluminiscent, où s'applique un polymère selon l'une des revendications 1 à 12 sous la forme d'un film sur un substrat.

16. Dispositif d'électroluminiscence avec l'une ou plusieurs couches, où au moins l'une de ces couches actives contient un polymère selon l'une des revendications 1 à 12.
